(19) **Europäisches Patentamt / European Patent Office / Office européen des brevets**

(11) **EP 3 094 382 B1**

(12) **EUROPEAN PATENT SPECIFICATION**

(45) Date of publication and mention of the grant of the patent:
**20.02.2019 Bulletin 2019/08**

(51) Int Cl.:
*A61Q 11/00* (2006.01)     *A61K 8/81* (2006.01)
*A61K 8/25* (2006.01)

(21) Application number: **14824809.9**

(22) Date of filing: **17.12.2014**

(86) International application number:
**PCT/EP2014/078200**

(87) International publication number:
**WO 2015/106915 (23.07.2015 Gazette 2015/29)**

(54) **ORAL CARE COMPOSITIONS**

MUNDPFLEGEMITTEL

COMPOSITIONS DE PROTECTION ORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2014 EP 14151161**

(43) Date of publication of application:
**23.11.2016 Bulletin 2016/47**

(73) Proprietors:
• **Unilever PLC**
  **London Greater London EC4Y 0DY (GB)**
  Designated Contracting States:
  **CY GB IE MT**
• **Unilever NV**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
• **ASHCROFT, Alexander, Thomas**
  **Wirral**
  **Merseyside CH63 3JW (GB)**

• **FERRY, Anne-Laure, Sophie**
  **Wirral**
  **Merseyside CH63 3JW (GB)**
• **GROVES, Brian, Joseph**
  **Wirral**
  **Merseyside CH63 3JW (GB)**
• **WILLIAMS, Adrian, Kevin, Norman**
  **Wirral**
  **Merseyside CH63 3JW (GB)**
• **WILSON, William, John**
  **Wirral**
  **Merseyside CH63 3JW (GB)**

(74) Representative: **Tansley, Sally Elizabeth**
  **Unilever PLC**
  **Unilever Patent Group**
  **Colworth House**
  **Sharnbrook**
  **Bedford**
  **Bedfordshire MK44 1LQ (GB)**

(56) References cited:
  **WO-A1-2004/047783     WO-A2-03/000217**
  **WO-A2-2010/037701     WO-A2-2011/094499**
  **FR-A1- 2 755 010       US-A1- 2007 122 359**

**Description**

**Field of the Invention**

The present invention is concerned with oral care compositions containing a polymeric structurant. Abrasives for use in oral care compositions such as dentifrices, are required to be effective in removing extrinsic stains, dental plaque and food debris which builds up on the pellicle on the surface of teeth.

WO2010/037710 discloses dentifrice composition comprising a source of fluoride ions a xanthan gum, a polyacrylic acid, from 0.01-2.0 percent a carageenan gum and a thickening silica.

US 2007/122359 discloses a dentifrice composition comprising; a binder system comprising a hydrophilic clay material, a modified cellulose polymer, a carboxyvinyl polymer and a natural gum derived anionic polymer.Abrasives for use in oral care compositions such as dentifrices, are required to be effective in removing extrinsic stains, dental plaque and food debris which builds up on the pellicle on the surface of teeth.

In general, the efficiency of physical removal of stain, plaque and food debris can be increased by using an abrasive having increased abrasivity, or by increasing the level of abrasive incorporated into the composition. However, both these approaches also increase the risk that tooth surfaces may be damaged.

Accordingly, there is a continuing need for oral care compositions that demonstrate satisfactory levels of cleaning, yet are not unduly abrasive and damaging to the teeth. The present inventors have found that the cleaning efficiency of an oral care composition comprising particulate abrasive may be surprisingly enhanced by the inclusion of specific structurants.

The present invention provides an oral care composition comprising:

i) an aqueous continuous phase including at one or more polyhydric alcohols;

ii) a silica particulate abrasive;

iii) from 0.05 to 1.0 wt% of the total composition of carbopol and optionally an additional structurant which is xanthan gum, in which in which the weight ratio of carbopol to additional structurant is greater than 5:1,

Compositions of the invention comprise carbomer. Suitable carbomer includes Carbopol 2984, Carbopol 934, Carbopol Ultrez 10, Carbopol 981 and Carbopol 2020 (EX Lubrizol Corporation. The level of carbopol is from 0.05 to 1.0 wt% of the total composition, more preferably from, 0.1 to 0.6 wt%, most preferably from 0.15 to 0.4 wt%.

Furthermore, the oral care composition, especially in the cases of dentifrices, dentifrice may also contain additional structurants agents. Such other binders or thickening agents will generally be present in an amount of in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth). If present the level of additional structurants is such that the weight ratio of carbopol to additional stucturant is greater than 1:1, more preferably greater than 2:1, most preferably greater than 5:1. If the additional structurant is xanthan gum the weight ratio of carbopol to xanthan gum is greater than 5:1,

Such ratios are particularly advantageous if the additional structurant is xanthan gum. In a preferred embodiment carbopol is the only structurant present in the composition.

Examples of suitable product forms for compositions of the invention include dentifrices, mouthwashes, mouthsprays and liquid formulations, such as paints or lacquers, which are designed to be applied directly to the tooth surface using an applicator such as a brush or sponge tip applicator.

Preferred product forms for compositions of the invention are those which are suitable for brushing and/or rinsing the surfaces of the oral cavity.

In such preferred product forms, the amount of water and/or polyhydric alcohol will generally be at least 10 percent, preferably at least 30 percent, more preferably at least 50 percent by total weight water and/or polyhydric alcohol based on the total weight of the composition.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. In general, a dentifrice may take various physical forms such as solid, semi-solid or liquid (or a combination thereof). For the purposes of this invention the dentifrice is preferably in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The oral care composition, in particular dentifrice compositions comprises silica abrasive materials. Abrasive materials will generally be present in an amount of from 0.5 to 75%, preferably 3 to 60 by weight based on the total weight of the dentifrice. Further suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Compositions of the invention may also comprise thickeners such as finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

Therefore the invention also provides a method for cleaning the surfaces of the oral cavity comprising the following steps:

a) topically applying the composition according to the invention to the oral cavity surfaces to be cleaned;
b) agitating the composition over the oral cavity surfaces by brushing and/or rinsing.

The invention is further illustrated with reference to the following, non-limiting Examples. Numbered Examples represent formulations according to the invention. Lettered Examples are comparative examples (not according to the invention).

## Examples

Table 1

| Ingredient | Use | Example A | Example B | Example C | Example 1 |
|---|---|---|---|---|---|
| Zeodent 113 | Abrasive silica | 13 | 13 | 13 | 13 |

(continued)

| Ingredient | Use | Example A | Example B | Example C | Example 1 |
|---|---|---|---|---|---|
| Zeodent 165 | Thickening silica | 6 | 6 | 6 | 6 |
| Carrageenan | Structurant | 0.5 | 1.5 | 0 | 0 |
| Xanthum gum | Structurant | 1 | 0 | 1.25 | 0 |
| Carbomer 2984 | Structurant | 0 | 0 | 0.2 | 0.2 |
| Steareth-30 | Surfactant | 3.0 | 3.0 | 3.0 | 3.0 |
| Sodium Saccharin | Sweetener | 0.1 | 0.1 | 0.1 | 0.1 |
| Titanium dioxide | Opacifier | 0.5 | 0.5 | 0.5 | 0.5 |
| Citric acid | Buffer | 0.28 | 0.28 | 0.28 | 0.28 |
| Disodium Phosphate | Buffer | 0.6 | 0.6 | 0.41 | 0.41 |
| Sodium Phosphate | Buffer | 0 | 0 | 1.0 | 1.0 |
| Glycerin | Solvent | 5.0 | 5.0 | 5.0 | 5.0 |
| Sorbitol | Solvent | 28.0 | 28.0 | 28.0 | 28.0 |
| Flavour | Flavour | 0.7 | 0.7 | 0.7 | 0.7 |
| Sodium Benzoate | Preservative | 0.15 | 0.15 | 0.15 | 0.15 |
| Water | Solvent | To 100 | To 100 | To 100 | To 100 |

The PCR was measured for each of the above formulations. PCR was measured using a method based on that described by Pickles et al. (International Dental Journal 55 (2005), pp. 197-202). This model uses enamel slabs cut from bovine central incisors, embedded in methacrylate resin. The enamel surfaces are smoothed by hand using an alumina paste on a glass block and lightly acid etched in order to facilitate stain accumulation and adherence. The enamel blocks are placed in an incubator set at a constant temperature of 50°C and slowly rotated to alternate between immersion in a staining broth (consisting of tea, coffee and mucin) and air drying. The broth is changed daily and after five days the slabs are removed and washed with distilled water to remove any loose debris. The stained slabs are then brushed in a mechanical brushing machine with distilled water to remove any loosely adhered stain. They are then dried and the L* values ($L^*_{stained}$) from the CIE L*a*b* colour system are measured with a chroma meter in L*a*b* mode. To assess cleaning performance, the stained specimens are then mounted in the mechanical brushing machine and the required load applied to each brush. The test composition is dispersed in an aqueous diluent to form a slurry (typically 38.5g test composition and 61.5g of distilled water) and the stained specimens brushed for a set number of brush strokes with 10ml of slurry. After brushing, the enamel slabs are rinsed with distilled water, dried and the L* values ($L^*_{brushed}$) are re-measured. In the final stage, all traces of stain are removed from the enamel slabs using flour of pumice, on a soft cloth using a grinder/polisher. The enamel slabs are then rinsed with distilled water, dried and the L* values ($L^*_{pumiced}$) are measured and recorded. The percentage of the stain removed by the test composition compared to full removal by pumice (hereinafter referred to as the pellicle cleaning ratio or PCR) may be calculated using the following equation:

$$PCR = [(L^*_{brushed})-(L^*_{stained})/ (L^*_{pumiced})-(L^*_{stained})] \times 100$$

The PCR results after 800 brush strokes are shown in Table 2 below.

**Table 2**

| Test Formulation | Mean 800 (s.d.) |
|---|---|
| Example A | 30.93(4.55) |
| Example B | 19.52(3.64) |
| Example C | 28.21(5.71) |

(continued)

| Test Formulation | Mean 800 (s.d.) |
|---|---|
| Example 1 | 53.00 (8.90) |

It can be seen from these results in table 2 that the cleaning performance a dentifrice comprising carbopol is surprisingly enhanced, versus the control examples A to C. It should be noted that example C comprises carbopol but also includes xanthum gum as a structurant at a level outside the required ratio of xanthum gum to carbopol, the inclusion of xanthum gum at this level is detrimental to cleaning enhancement caused by the carbopol.

Formulations with differing levels of carbopol are given in Table 3.

**Table 3**

| Ingredient | Use | Ex. D | Ex. 2 | Ex. 3 | Ex. 4 | Ex. 5 | Ex. 6 |
|---|---|---|---|---|---|---|---|
| Sorbosil AC77 | Abrasive silica | 10 | 10 | 10 | 10 | 10 | 10 |
| Sorbosil TC15 | Thickening silica | 9.5 | 9.5 | 9.5 | 9.5 | 9.5 | 9.5 |
| Carbomer 2984 | Structurant | 0 | 0.1 | 0.2 | 0.3 | 0.4 | 0.5 |
| Pluriol flakes | Humectant | 2.0 | 2.0 | 2.0 | 2.0 | 2.0 | 2.0 |
| Sodium Saccharin | Sweetener | 0.25 | 0.25 | 0.25 | 0.25 | 0.25 | 0.25 |
| Titanium dioxide | Opacifier | 1.0 | 1.0 | 1.0 | 1.0 | 1.0 | 1.0 |
| SCMC 9H | Structurant | 0.6 | 0.6 | 0.6 | 0.6 | 0.6 | 0.6 |
| Sodium Fluoride | Active | 0.32 | 0.32 | 0.32 | 0.32 | 0.32 | 0.32 |
| Empicol LZN | Surfactant | 1.8 | 1.8 | 1.8 | 1.8 | 1.8 | 1.8 |
| Neosorb 70/70 B | Polyol (Sorbitol) | 45.0 | 45.0 | 45.0 | 45.0 | 45.0 | 45.0 |
| Flavour | Flavour | 1.1 | 1.1 | 1.1 | 1.1 | 1.1 | 1.1 |
| Water | Solvent | To 100 | To 100 | To 100 | To 100 | To 100 | To 100 |

The PCR was measured as described above and the results shown in Table 4.

**Table 4**

| Test Formulation | Mean 800 (s.d.) |
|---|---|
| Example D | 18.05(4.21) |
| Example 2 | 24.28(2.49) |
| Example 3 | 28.85(7.72) |
| Example 4 | 28.99(6.49) |
| Example 5 | 30.43(8.22) |
| Example 6 | 29.88(5.02) |

It can be seen from the results above the carbopol enhances the cleaning, however the cleaning efficacy plateaus at levels of carbopol above 0.4 wt%.

**Claims**

1. An oral care composition comprising:

   i) an aqueous continuous phase including at one or more polyhydric alcohols;
   ii) a silica particulate abrasive

iii) from 0.05 to 1.0 wt% of the total composition of carbopol and optionally an additional structurant which is xanthan gum, in which the weight ratio of carbopol to additional structurant is greater than 5:1,

2. An oral care composition according to any preceding claim in which the level of xanthan gum is from 0 to 0.5 wt% of the total composition.

3. An oral care composition according to any preceding claim in which the level of polyhydric alcohol is from 20 to 60 wt% of the total composition.

4. An oral care composition according to any preceding claim which comprises from 10 to 50 wt% of the total composition of water.

5. An oral care composition according to any preceding claim which is in the form of a dentifrice.

6. An oral care composition according to any preceding claim, which further comprises a surfactant in an amount of from 0.2 to 5 wt% of the total composition.

7. An oral care composition according to any preceding claim in which the polyhydric alcohol is sorbitol.

**Patentansprüche**

1. Mundpflegemittel, umfassend:

   i) eine wässrige kontinuierliche Phase, die einen oder mehr mehrwertige Alkohole enthält,
   ii) ein teilchenförmiges abrasives Siliciumdioxid,
   iii) von 0,05 bis 1,0 Gew.-% der gesamten Zusammensetzung Carbopol und optional ein zusätzliches Strukturierungsmittel, das Xanthangummi ist, in welchem das Gewichtsverhältnis von Carbopol zu zusätzlichem Strukturierungsmittel größer als 5:1 ist.

2. Mundpflegemittel nach irgendeinem vorhergehenden Anspruch, in welchem der Anteil an Xanthangummi von 0 bis 0,5 Gew.-% der gesamten Zusammensetzung beträgt.

3. Mundpflegemittel nach irgendeinem vorhergehenden Anspruch, in welchem der Anteil des mehrwertigen Alkohols von 20 bis 60 Gew.-% der gesamten Zusammensetzung beträgt.

4. Mundpflegemittel nach irgendeinem vorhergehenden Anspruch, welches von 10 bis 50 Gew.-% der gesamten Zusammensetzung an Wasser umfasst.

5. Mundpflegemittel nach irgendeinem vorhergehenden Anspruch, welches in Form eines Zahnputzmittels vorliegt.

6. Mundpflegemittel nach irgendeinem vorhergehenden Anspruch, welches ferner ein Tensid in einer Menge von 0,2 bis 5 Gew.-% der gesamten Zusammensetzung umfasst.

7. Mundpflegemittel nach irgendeinem vorhergehenden Anspruch, in welchem der mehrwertige Alkohol Sorbitol ist.

**Revendications**

1. Composition pour le soin oral comprenant :

   i) une phase continue aqueuse incluant un ou plusieurs alcools polyvalents ;
   ii) un abrasif particulaire de silice
   iii) de 0,05 à 1,0 % en masse de la composition totale de carbopol et éventuellement d'un structurant supplémentaire qui est de la gomme xanthane, dans laquelle le rapport massique de carbopol à structurant supplémentaire est supérieur à 5:1.

2. Composition pour le soin oral selon l'une quelconque des revendications précédentes, dans laquelle la teneur de gomme xanthane est de 0 à 0,5 % en masse de la composition totale.

3. Composition pour le soin oral selon l'une quelconque des revendications précédentes, dans laquelle la teneur d'alcool polyvalent est de 20 à 60 % en masse de la composition totale.

4. Composition pour le soin oral selon l'une quelconque des revendications précédentes qui comprend de 10 à 50 % en masse de la composition totale d'eau.

5. Composition pour le soin oral selon l'une quelconque des revendications précédentes qui est dans la forme d'un dentifrice.

6. Composition pour le soin oral selon l'une quelconque des revendications précédentes, qui comprend de plus un tensioactif dans une quantité de 0,2 à 5 % en masse de la composition totale.

7. Composition pour le soin oral selon l'une quelconque des revendications précédentes, dans laquelle l'alcool polyvalent est le sorbitol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010037710 A
- US 2007122359 A

**Non-patent literature cited in the description**

- **PICKLES et al.** *International Dental Journal,* 2005, vol. 55, 197-202